# DEMANDE DE BREVET EUROPEEN

(11) **EP 2 567 670 A1**
(43) Date de publication de la demande: **13.03.2013**
(21) Numéro de dépôt: 12183554.0
(22) Date de dépôt: 07.09.2012
(51) Int. Cl.: A61B 19/00, A61M 25/01

(54) **Module et procédé d'entraînement d'organes médicaux souples allongés et système robotisé associé**

(30) Priorité: 07.09.2011 FR 1157942
(71) Demandeur: Robocath, 76000 Rouen (FR)
(72) Inventeur: Bencteux, Philippe, 76230 Bois-Guillaume (FR); Deboeuf, Sébastien, 95220 Herblay (FR); Marignier, Jacques, 76240 Le Mesnil Esnard (FR)
(74) Mandataire: Cabinet Plasseraud

(57) **Abrégé**

Module d'entraînement d'un organe médical souple allongé selon une première direction, qui comprend :
- un canal (49),
- de chaque côté du canal :
. une première et une deuxième poulies (31a, 31d) comportant une surface d'entraînement,
. une bande (61a, 61b) allongée comprenant une première face et une deuxième face opposée, la première face coopérant avec la surface d'entraînement des poulies, la deuxième face coopérant avec l'organe médical souple, la bande étant tendue par les poulies avec une portion allongée s'étendant dans le canal selon la première direction,

au moins une des poulies étant motorisée.

## Description

La présente invention est relative aux modules et procédés d'entraînement d'organes médicaux souples allongés, et aux systèmes robotisés associés.

Plus particulièrement, l'invention se rapporte à un module d'entraînement d'un organe médical souple allongé selon une première direction, comprenant une base et un équipage mobile monté rotatif sur ladite base autour de la première direction, l'équipage mobile comportant un support.

Un exemple typique d'organe médical souple allongé est par exemple un cathéter. Un tel cathéter doit être introduit dans un conduit anatomique d'un patient, et doit donc être relativement souple. L'extrémité du cathéter doit aussi parvenir jusqu'à un organe interne du patient, il doit donc être relativement allongé. D'autres exemples d'organes médicaux souples allongés sont par exemple un guide, qui est de diamètre inférieur, et généralement disposé à l'intérieur du cathéter et sur lequel ce dernier glisse, ou un cathéter interventionnel, également disposé à l'intérieur du cathéter, et dont l'extrémité fournit une certaine fonction médicale telle qu'un outil médical (pince, ballon, etc...).

L'insertion de tels cathéters est généralement monitorée sous rayons X. Il en découle une irradiation certaine du médecin pratiquant de manière répétée de telles insertions.

Des efforts ont été faits pour robotiser cette insertion. Ainsi, la manipulation du cathéter est assurée par le robot, qui est commandé à distance par le médecin, toujours sous guidage aux rayons X, mais dans une pièce non irradiée.

WO 2009/137,410 décrit de nombreux exemples de réalisation, illustratif de tels efforts. Toutefois, ces systèmes, très complexes, ne sont pas bien adaptés à une mise en oeuvre en milieu hospitalier, par du personnel qui n'est pas qualifié techniquement. De plus, la dissociation de l'entraînement en translation et de l'entraînement en rotation du cathéter rend la mise en oeuvre d'un tel système hasardeuse.

Le document US 7,927,310 décrit un exemple d'un système selon le préambule de la revendication 1.

Ce système présente le grand avantage d'être bien adapté au caractère stérile des cathéters ou autres organes introduits dans le patient, qui baignent dans un liquide de conservation tel que du sérum physiologique. Toutefois, dans ce document, le cathéter est entrainé, au niveau du module d'entraînement par 2 roues disposées d'un même côté, une troisième roue, non-entrainée, disposée de l'autre côté du cathéter servant à maintenir la pression.

En pratique, il a été observé des problèmes d'entraînement du cathéter avec un tel mécanisme. Ces problèmes sont notamment dus d'une part à la souplesse du cathéter, et d'autre part à sa conservation en milieu liquide.

La présente invention a notamment pour but de pallier ces inconvénients.

A cet effet, selon l'invention, un module d'entraînement du genre en question est caractérisé en ce que le module comprend :
- un canal défini dans le support, et s'étendant selon la première direction,
- de chaque côté du canal :
   . au moins une première et une deuxième poulies comportant une surface d'entraînement, et portées par le support,
   . une bande allongée comprenant une première face et une deuxième face opposée, la première face coopérant avec la surface d'entraînement des poulies, la deuxième face adaptée pour coopérer avec l'organe médical souple, la bande étant tendue par les poulies avec une portion allongée s'étendant dans le canal selon la première direction,
au moins une des poulies étant motorisée.

Grâce à ces dispositions, on optimise la linéarité du cathéter au niveau du module d'entraînement, ce qui permet d'améliorer son entraînement.

Dans des modes de réalisation préférés de l'invention, on peut éventuellement avoir recours en outre à l'une et/ou à l'autre des dispositions suivante :
- le module comprend en outre un dispositif de réglage placé entre la première et la deuxième poulie, une surface tendeuse coopérant avec la première face de la bande, et mobile par rapport au canal le long d'une direction d'ajustement transverse à la première direction ;
- la première face de la bande présente des dents d'entraînement et la surface d'entraînement de la poulie motorisée présente des dents complémentaires auxdites dents d'entraînement ;
- le module comprend un ensemble de pièces d'entrainement coopérant entre elles pour transmettre un mouvement d'un moteur à la poulie motorisée ;
- l'équipage mobile est monté rotatif sur la base autour d'un premier axe, et le canal comprend une portion définie dans le support selon un deuxième axe, parallèle au premier axe et décalé par rapport à celui-ci ;
- le module comprend un dispositif de latéralisation adapté pour régler un décalage du premier et du deuxième axe ;
- le dispositif de latéralisation comporte une commande adaptée pour, lors d'une première partie de course d'entraînement, causer un enserrement de l'organe médical, et lors d'une deuxième partie de course d'entraînement suivant la première partie, régler ledit décalage du premier et du deuxième axe ;
- le module est constitué desdites bandes, de pièces stérilisables, et de pièces d'assemblage amovibles à déformation élastique adaptées pour maintenir ensemble les pièces stérilisables avec liberté de mouvement les unes par rapport aux autres.

Selon un autre aspect, l'invention se rapporte à un système robotisé qui comprend :
- un tel module d'entraînement,
- un récipient adapté pour contenir un organe médical souple allongé en condition aqueuse stérile, et en communication avec ledit canal,
- un moteur coopérant avec la poulie motorisée.

Selon un mode de réalisation, le système comprend en outre une barrière stérile, le moteur coopérant avec la poulie motorisée à travers la barrière stérile.

Selon un autre aspect, on prévoit un procédé d'entraînement d'un organe médical souple allongé selon une première direction, qui comprend :
- on fournit un module d'entraînement comprenant :
   . un canal s'étendant selon la première direction,
   . de chaque côté du canal :
      .. au moins une première et une deuxième poulies comportant une surface d'entraînement,
      .. une bande allongée comprenant une première face et une deuxième face opposée, la première face coopérant avec la surface d'entraînement des poulies, la deuxième face adaptée pour coopérer avec l'organe médical souple, la bande étant tendue par les poulies avec une portion allongée s'étendant dans le canal selon la première direction,
- on place un organe médical souple allongé selon la première direction partiellement dans ledit canal,
- on motorise au moins une des poulies.

Selon un autre aspect, l'invention se rapporte à un système d'artériographie robotisé comprenant :
- une base munie d'un premier organe de transmission,
- un module d'entraînement d'un organe médical flexible allongé selon une direction d'extension comprenant :
   . un deuxième organe de transmission adapté pour coopérer avec le premier organe de transmission,
   . au moins un organe d'entraînement raccordé au deuxième organe de transmission, et adapté pour coopérer avec un organe médical flexible allongé pour alternativement maintenir ledit organe médical fixe par rapport à la base ou translater ledit organe médical selon la direction d'extension,
- une barrière de stérilité recouvrant la base, les premier et deuxième organes de transmissions coopérant à travers la barrière de stérilité,
dans lequel le module d'entraînement est constituée d'un ensemble de pièces consommables, d'un ensemble de pièces stérilisables, et d'un ensemble de pièces d'assemblage à déformation élastique, lesdites pièces stérilisables étant maintenues les unes aux autres avec liberté de mouvement par lesdites pièces d'assemblage.

Selon un autre aspect, une invention se rapporte à un système de cathétérisme robotisé comprenant :
- une unité centrale,
- au moins une première, une deuxième et une troisième lignes de commande adaptées respectivement pour envoyer une commande à un premier, un deuxième et un troisième moteurs d'entraînement d'un même cathéter,
- dans lequel les première et deuxième lignes de commande sont commandées selon un premier rapport prédéfini pour commander un mouvement de translation pure du cathéter le long de son axe, et dans lequel les première et troisième lignes de commande sont commandées selon un deuxième rapport prédéfini pour commander un mouvement de rotation pure du cathéter le long de son axe.

Selon un autre aspect, une invention se rapporte à un module d'entraînement comprenant une base et un équipage mobile monté rotatif sur ladite base autour de la première direction, dans lequel l'équipage mobile comprend :
- une partie de fond comprenant au moins un arbre d'entraînement, et
- une partie consommable dans laquelle ledit canal est défini, la partie consommable comprenant au moins lesdites poulies, la partie consommable étant assemblable sur la partie de fond, la poulie motorisée coopérant avec ledit arbre d'entraînement.

La partie consommable sera jetée après utilisation, et remplacée par une pièce identique pour une utilisation future.

Selon un autre aspect, une invention se rapporte à un module d'entraînement d'un organe médical souple allongé selon une première direction, comprenant une base et un équipage mobile monté rotatif sur ladite base autour d'un premier axe s'étendant selon la première direction, l'équipage mobile comportant un support,
dans lequel le module comprend :
- un canal défini dans le support, et s'étendant selon la première direction,
- de chaque côté du canal :
   . au moins un premier et un deuxième organe d'entraînement portés par le support, et présentant chacun une face adaptée pour coopérer avec l'organe médical souple,
dans lequel le canal comprend une portion selon un deuxième axe, parallèle au premier axe et décalé par rapport à celui-ci.

En particulier, l'amplitude du décalage d'axe peut être variable.

D'autres caractéristiques et avantages de l'invention apparaîtront au cours de la description suivante de plusieurs formes de réalisation, donnée à titre d'exemple non limitatif, en regard des dessins joints.

Sur les dessins :
- la figure 1 est une vue schématique d'une installation d'artériographie robotisée,
- la figure 2 est une vue éclatée partielle en perspective d'un robot de cathéterisme,
- la figure 3 est une vue en perspective d'un module d'entraînement pour le robot de la figure 2,
- la figure 4 est une vue de dessus du module de la figure 3 après ouverture du couvercle,
- la figure 5 est une vue éclatée en perspective de l'équipage mobile du module des figures 3 et 4,
- la figure 6 est une vue éclatée en perspective du module de la figure 3, et
- les figures 7a et 7b sont des vues de dessus du robot dans deux positions différentes,
- la figure 8 présente un agrandissement de la figure 2 montrant l'emplacement d'une barrière stérile,
- la figure 9 est une vue en coupe selon la ligne IX-IX de la figure 5,
- la figure 10 est une vue schématique en perspective en éclaté d'un deuxième mode de réalisation de plaquette,
- la figure 10a est une vue similaire à la figure 10 pour une variante de réalisation,
- les figures 11a, 11b et 11c illustrent divers états selon la commande appliquée aux différents moteurs,
- la figure 12 illustre un troisième mode de réalisation, et
- la figure 13 est une vue partielle schématique de face illustrant un quatrième mode de réalisation.

Sur les différentes figures, les mêmes références désignent des éléments identiques ou similaires.

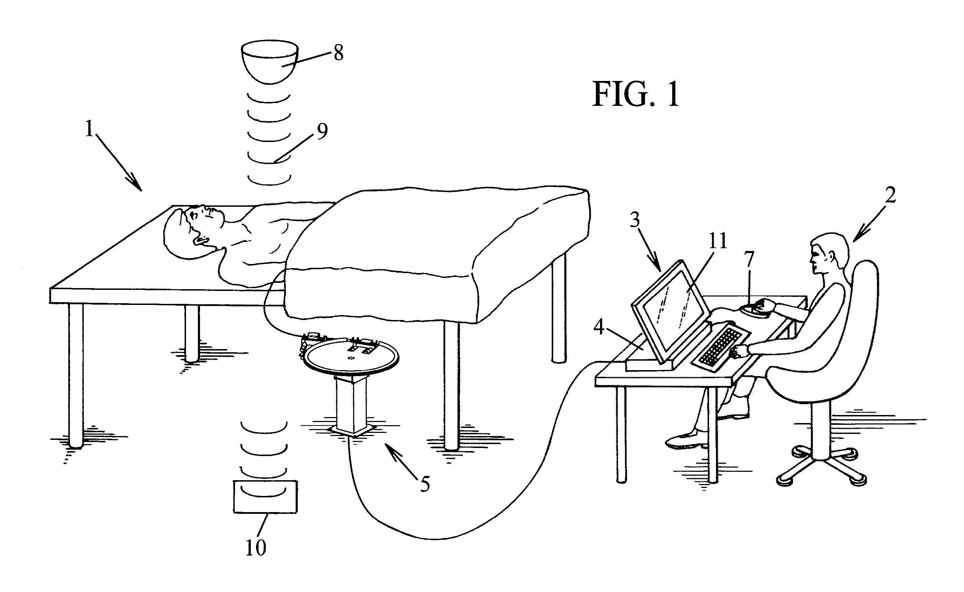

Sur la figure 1, un patient 1 est soumis à une artériographie. Cette artériographie est mise en oeuvre par un personnel 2, tel que par exemple un chirurgien, ou du personnel médical qualifié, utilisant un système d'artériographie 3 automatisé qui comprend par exemple une machine programmable 4 commandant à distance un enrouleur/dérouleur 5 à cathéters disposé à proximité du patient 1. L'enrouleur/dérouleur 5 est robotisé et est parfois également appelé « le robot ».

Le déplacement d'un cathéter 6 à l'intérieur du corps du patient 1 est commandé à distance par le personnel qualifié 2 à l'aide d'un moyen de commande 7 tel que par exemple une souris ou un joystick, reliée à la machine programmable 4.

L'installation comprend en outre une source de rayons X 8 émettant des rayons X 9 vers le patient 1, et un détecteur de rayons X 10 apte à détecter le rayonnement traversant le patient 1. Le détecteur 10 peut être relié à l'ordinateur 4 pour afficher sur l'écran 11 de celui-ci une image détectée.

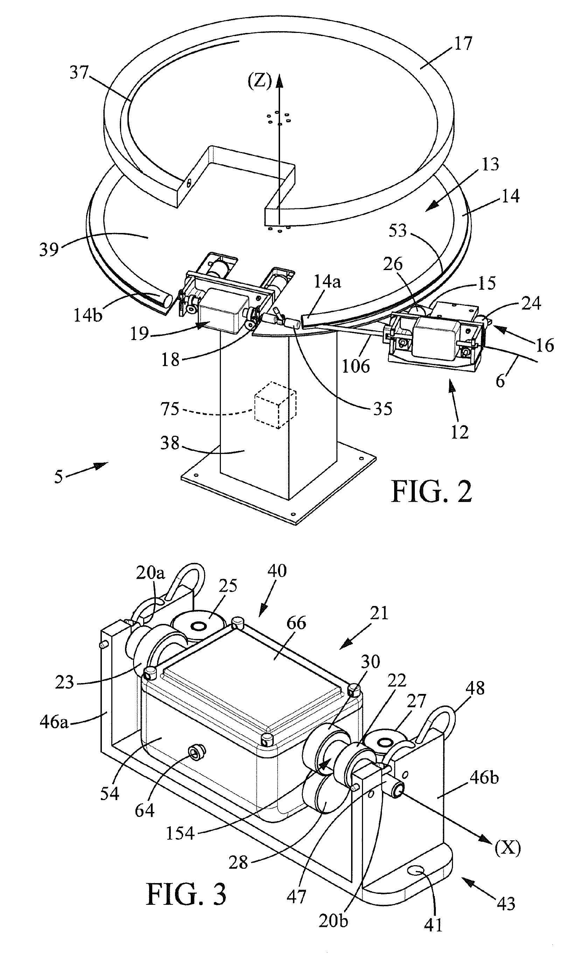

Comme représenté sur la figure 2, le robot 5 comporte un pied 38 vertical, un plateau 39 horizontal porté par ce pied, et au moins un premier système 12 et un deuxième système 13 concentriques disposés autour d'un axe par exemple orienté verticalement selon Z.

Le premier système 12 comporte un premier récipient 14 en forme de tube souple porté par le plateau à sa périphérie, par exemple clipsable dans des crochets portés par le plateau. Le tube souple s'étend d'une première extrémité 14a à une deuxième extrémité 14b faisant moins d'un tour du plateau. Le tube 14 reçoit un cathéter tubulaire 6 destiné à être introduit dans une artère du patient 1. Le tube souple 14 comprend une fente longitudinale 53 par laquelle le cathéter 6 peut entrer ou sortir du tube 14. Le tube 14 souple pourrait être remplacé par un tube rigide muni d'une lèvre pour l'entrée/la sortie du cathéter. Un tube de guidage rigide 106 s'étend entre la fente longitudinale 53 et le mécanisme d'entraînement 16. Il reçoit le cathéter 6 en son intérieur. La longueur du tube 14 est supérieure à la longueur classique d'un cathéter. Ce cathéter s'étend d'une première extrémité à une deuxième extrémité le long d'une direction d'élongation de cathéter. Le tube 14 est par ailleurs rempli d'un liquide de conservation dans lequel baigne le cathéter. Le premier système 12 comporte également un mécanisme d'entraînement 16, ou « module », qui sera décrit plus en détail par la suite en relation avec les figures 3 à 6. Ce mécanisme d'entraînement est porté par un bras 15 relié de manière fixe au pied, et est destiné à être placé à proximité immédiate de l'orifice du patient à cathétériser. La position du bras 15, notamment sa hauteur par rapport au sol, peut le cas échéant être ajustée pour les besoins de l'opération. Le mécanisme d'entraînement est commandé par le chirurgien 2 par l'intermédiaire de l'ordinateur 4.

Le deuxième système 13 comporte un deuxième récipient 17, destiné à être reçu fixé sur le plateau 39. Le deuxième système comporte également une sortie 18 par laquelle un guide 37 contenu dans le deuxième récipient peut accéder à l'extérieur du deuxième récipient, et en particulier à l'intérieur du premier récipient 14. En particulier, il accède à l'intérieur du cathéter compris dans le premier récipient.

Le deuxième récipient reçoit un guide tubulaire 37 s'étendant entre une première et une deuxième extrémité le long d'une direction d'élongation de guide, et baignant dans un liquide adapté à sa conservation. Par ailleurs, le deuxième récipient comporte également un mécanisme d'entraînement 19, similaire au premier mécanisme d'entraînement 16 du premier système 12, et qui sera décrit par la suite plus en détail en relation avec les figures 3 à 6.

Les figures 3 à 8 représentent le premier mécanisme d'entraînement 16 selon un exemple illustratif de réalisation. Le deuxième mécanisme d'entraînement 19 pourra être réalisé de manière similaire. Le premier mécanisme d'entraînement 16 comporte une base 43 fixée sur le bras 15. A titre d'exemple, le bras 15 porte une plaque support de fixation 42 percée de deux trous de fixation, et de deux trous de motorisation, et la base 43 présente une plaque percée de trous correspondants : trous de fixation 41 et trous de motorisation 50 placés en regard des trous de la plaque 42. Une barrière de stérilité 44 s'étend entre la plaque 42 associée au bras 15 et la base 43, en étant percée uniquement pour laisser passer à travers ces ouvertures les dispositifs de fixation 45 associés aux trous de fixation, et les arbres moteurs associés aux trous de motorisation 50. Ainsi, la barrière de stérilité 44 va recouvrir le bras 15, et notamment les moteurs 24 et 26, à l'exception de la base 43 et de ce qu'elle porte. La base 43 porte un équipage mobile 40 monté rotatif par rapport à la base 43 autour d'un axe de rotation confondu avec l'axe X d'élongation du cathéter 6 dans deux paliers 20a, 20b de la base. La base 43 présente un profil en U, où les deux bras parallèles du U 46a, 46b sont utilisés pour définir les paliers. Notamment le bras 46b comporte une fente 47 ouverte vers le haut, définissant une partie de palier, et refermable pour compléter le palier par un dispositif amovible 48 telle qu'une goupille. Une partie de la goupille et de la fente définissent ensemble le palier. Une réalisation similaire est prévue sur l'autre bras. L'équipage mobile comporte, dans l'exemple présenté, une plaquette 21 qui sera décrite plus en détail par la suite, portant le cathéter, une bague d'engrenage de rotation 23 cylindrique montée sur un arbre cylindrique amont 49, s'étendant selon l'axe X, et une bague d'engrenage de translation montée sur un arbre cylindrique aval 149, s'étendant selon l'axe X, à l'intérieur desquels passe le cathéter 6. Les arbres amont 49 et aval 149, alignés, définissent l'axe d'entraînement en translation et en rotation du cathéter.

Le premier mécanisme d'entraînement 16 comporte un moteur électrique de rotation 24 (figure 2) destiné à entraîner la rotation du cathéter autour de l'axe X. Le moteur 24 entraîne en rotation, lors du passage d'un courant électrique, sur la commande provenant de l'ordinateur 4, la rotation d'un arbre de rotation 51 portant engrenage de rotation 25 qui coopère avec la bague d'engrenage de rotation 23 de l'équipage mobile. Il est à noter que les engrenages 25 et 23 comportent des dents inclinées pour que la rotation de l'engrenage 25 autour d'un axe transverse à l'axe X puisse générer la rotation de l'engrenage 23 autour de l'axe X.

Le mécanisme d'entraînement 16 comporte également un moteur électrique de translation 26 (figure 2) destiné à entraîner la translation du cathéter le long de l'axe X. Le moteur 26 entraîne en rotation, sous l'effet du passage du courant électrique, sous commande de l'ordinateur 4, un arbre de translation 52 portant un engrenage de translation 27. Les arbres de translation et de rotation 51 et 52 sont parallèles. Un manchon 154 de transfert s'étend selon l'axe X et porte de manière décalée selon cet axe un engrenage de translation 22 et un engrenage intermédiaire 30. Les engrenages 27 et 22 présentent des dents inclinées pour que la rotation de l'engrenage 27 autour d'un axe transverse à l'axe X puisse générer la rotation de l'engrenage 22 autour de l'axe X.

Ces moteurs sont par exemple des moteurs pas à pas ou brushless.

La bague d'engrenage de translation 22 entraîne en rotation un arbre 28, monté rotatif dans deux paliers 29 internes à la plaquette 21 par l'intermédiaire d'un engrenage intermédiaire 30. L'arbre 28 porte une première et une deuxième portée dentée 28a, 28b. L'arbre 28 pourrait éventuellement être remplacé par une vis sans fin.

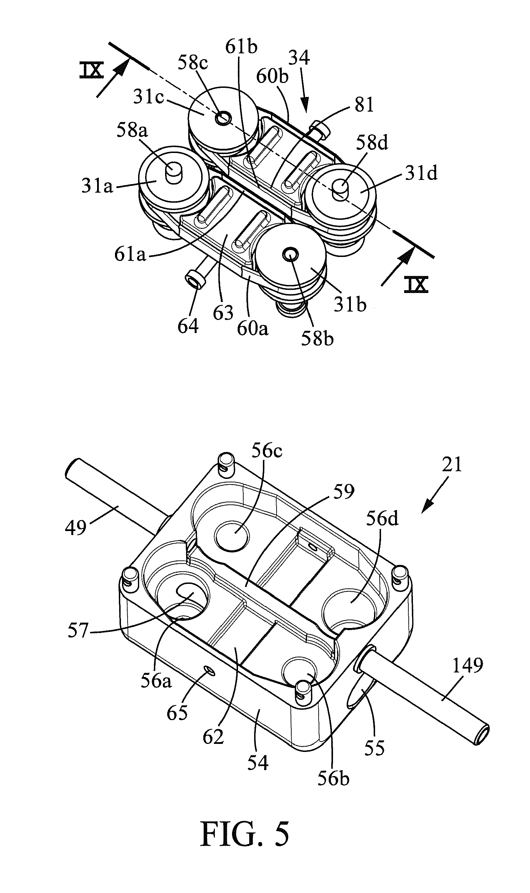

Comme cela est visible sur la figure 5, la plaquette 21 comporte une partie de fond 54 adaptée pour recevoir divers éléments. Un trou 55 cylindrique, s'étendant parallèlement à l'axe X, est visible, et définit les deux paliers 29 de réception de l'arbre 28.

Dans la plaquette sont ménagés quatre trous cylindriques 56a-56d s'étendant parallèlement les uns aux autres, dans une direction transversale à la direction X, et disposés de chaque côté de l'axe X. On notera que les trous cylindriques 56a et 56d opposés diagonalement débouchent dans le trou cylindrique 55 par le biais d'une ouverture respective 57.

Chacun des trous 56a-56d reçoit un arbre correspondant 58a-58d. Les arbres 58a et 58d sont identiques entre eux et comportent, au niveau de l'ouverture 57, une roue dentée adaptée pour coopérer avec la portée dentée correspondante de l'arbre 28, de sorte qu'une rotation de l'arbre 28 autour de son axe entraîne une rotation de l'arbre 58a ou 58d respectif autour de son axe.

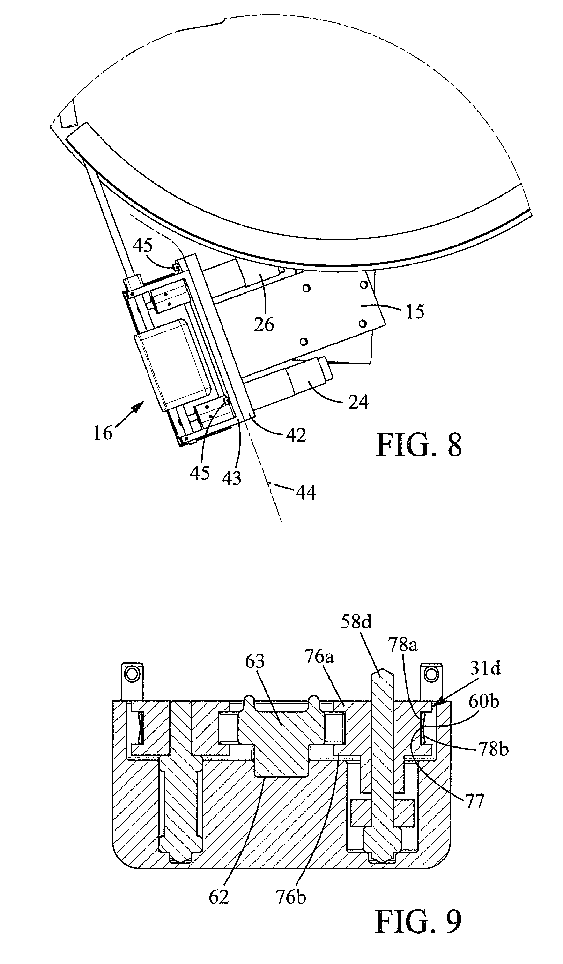

Chacun des arbres 58a-58d porte également une poulie 31a-31d respective. Comme visible sur la figure 9, la poulie 31d comprend deux disques superposés 76a, 76b, le long de l'arbre, entre lesquels s'étend une surface 77 d'entraînement de la courroie 60b. Les disques servent de butées supérieure et inférieure à la courroie. Une même description s'applique pour toutes les poulies. La courroie 60b comporte une face interne 78 dentée, coopérant avec la surface d'entraînement 77, et une face externe 78b opposée. La face externe 78b est destinée à coopérer avec le cathéter. Elle est constituée d'un matériau adapté, pouvant subir les déformations appliquées à la courroie lors de la motorisation, et restant solidaire du cathéter, même mouillé. On utilise par exemple un élastomère, caoutchouc ou silicone adapté. De retour sur la figure 5, un canal 59 est défini entre ces quatre poulies, et peut recevoir un cathéter. Les deux poulies 31a et 31b sont associées entre elles en ce qu'une courroie 60a est tendue entre ces deux poulies. De même, une courroie 60b est tendue entre les deux poulies 31c et 31d.

Dans une position donnée du module d'entraînement, chaque courroie comprend une portion de bande 61a et 61b s'étendant dans le canal, et en prise avec le cathéter passant dans le canal. Les parties de bandes sont allongées le long de la direction longitudinale, et courent dans cette direction en cas d'actionnement du moteur de translation.

Un mécanisme de réglage 34 permet de régler l'utilisation du module à l'organe souple allongé à entrainer. Ainsi, le mécanisme de réglage 34 peut être utilisé pour régler finement le système d'entraînement pour différents cathéters identiques, ou pour modifier largement la largeur du canal dans le cas où le module doit être adapté pour l'entraînement d'un cathéter ou d'un guide, de diamètre largement inférieur. Dans l'exemple présenté, le mécanisme de réglage 34 comprend une glissière longitudinale 62 formée dans la partie de fond 54 de la plaquette 21, et s'étendant transversalement à la direction longitudinale X, et un poussoir 63 adapté pour coulisser dans cette glissière 62 en direction de ou en s'éloignant du canal. Le poussoir 63 est disposé à l'intérieur de la périphérie de la courroie respective 60a ou 60b, et présente une surface tendeuse 81 qui coopère avec la face interne 78a de la courroie de manière à déplacer la partie de bande 61a ou 61b de la courroie transversalement à la direction X. La fixation du poussoir 63 dans la position réglée peut se faire par tout moyen approprié, notamment par une vis 64 vissée dans un alésage 65 ménagé dans la partie de fond 54. Le poussoir présente une longueur (selon l'axe de la glissière) supérieure au diamètre des poulies. Ainsi, quelle que soit la position du poussoir par rapport à la courroie dans la gamme de réglage, la tension des courroies reste sensiblement la même.

Les courroies présentent une surface interne 78a adaptée pour être entraînée par la poulie motrice, et pour glisser le long des surfaces, coopérant avec elle, du poussoir 63. Une telle surface interne est par exemple dentée. La surface externe 78b de la courroie est réalisée dans un matériau adapté pour coopérer avec l'organe médical allongé, souple et humide et par exemple peut comporter un matériau élastomère adapté.

En cours d'utilisation, un couvercle 66 est assemblé à la partie de fond 54 de la plaquette 21, afin de maintenir les différentes parties qui viennent d'être décrites dans la plaquette. Dans le présent exemple de réalisation, les arbres 58a et 58d sont moteurs et sont, entraînés, lors de la rotation de l'arbre 28, de sorte que les parties de bande 61a et 61b se déplacent au niveau du canal dans une même direction, pour une rotation donnée du moteur d'entraînement en translation. Ainsi, ces parties de bande appliquent directement le mouvement à l'organe souple allongé. Les arbres 58b, 58c sont menés, et ne sont pas directement entraînés par les moteurs de translation, mais par les courroies.

Comme représenté sur la figure 2, la sortie 18 du deuxième système comporte un dispositif de fixation 35 permettant la libre rotation du cathéter 6 autour de son axe d'élongation par rapport au deuxième système, et l'entraînement du deuxième récipient 17 lors de la translation du cathéter 6 le long de sa direction d'élongation. Selon un exemple purement illustratif du dispositif de fixation 35, l'extrémité distale du cathéter 6 est fixée sur une bague présentant une rainure qui s'insère dans un orifice complémentaire formé dans le deuxième récipient. La bague est ainsi libre de tourner par rapport au deuxième récipient autour d'un axe d'élongation du guide 37. La bague peut présenter une forme évasée vers le cathéter 6, pour faciliter sa préhension manuelle par un utilisateur.

Selon une variante, le volet pourrait être remplacé par un anneau ouvert rapporté sur le deuxième récipient.

On notera que le mode de réalisation présenté sur la figure 2 peut par exemple être réalisé modulaire, à savoir que le premier système 12 et le deuxième système 13 peuvent être commercialisés séparément et assemblés pour les besoins de l'examen du patient.

On peut d'ailleurs prévoir en outre, un troisième système insérable dans le deuxième système et concentrique autour de l'axe Z avec celui-ci, pour l'insertion dans le patient d'un cathéter interventionnel, en plus du guide et du cathéter. De manière similaire le troisième système comporte un récipient pouvant contenir un liquide, un mécanisme d'entraînement et une sortie débouchant dans le deuxième système. Les systèmes sont disposés de sorte que l'élément à insérer présentant le plus large diamètre (généralement le cathéter) est placé dans le système externe, et l'élément à insérer présentant le plus fin diamètre (généralement le guide) est placé dans le système interne, le cathéter interventionnel étant par conséquent placé dans le système intermédiaire.

En outre, chaque système peut lui-même être réalisé de manière modulaire, à savoir que les moteurs électriques peuvent être fournis à demeure sur le robot, et le reste du système peut en être démonté pour être remplacé par une pièce consommable sans aucun moteur électrique. De cette manière, on diminue grandement les coûts liés à la réutilisation du système, car on peut garder d'une utilisation à l'autre les éléments les plus coûteux (ceux-ci pouvant éventuellement être stérilisés ou décontaminés avant leur utilisation ultérieure).

La description ci-dessus a été donnée par référence au premier système d'entraînement 12. Une description similaire est possible pour le deuxième mécanisme d'entraînement 19. De manière similaire à la première barrière stérile 44, une deuxième barrière stérile 144, indépendante de la première, peut être glissée entre la plaque 42 et le module correspondant. La deuxième barrière stérile 144 va recouvrir les moteurs 24 et 26 du deuxième système et le plateau 39. Par contre, le tube 14 et le récipient 17 vont être placés au-dessus.

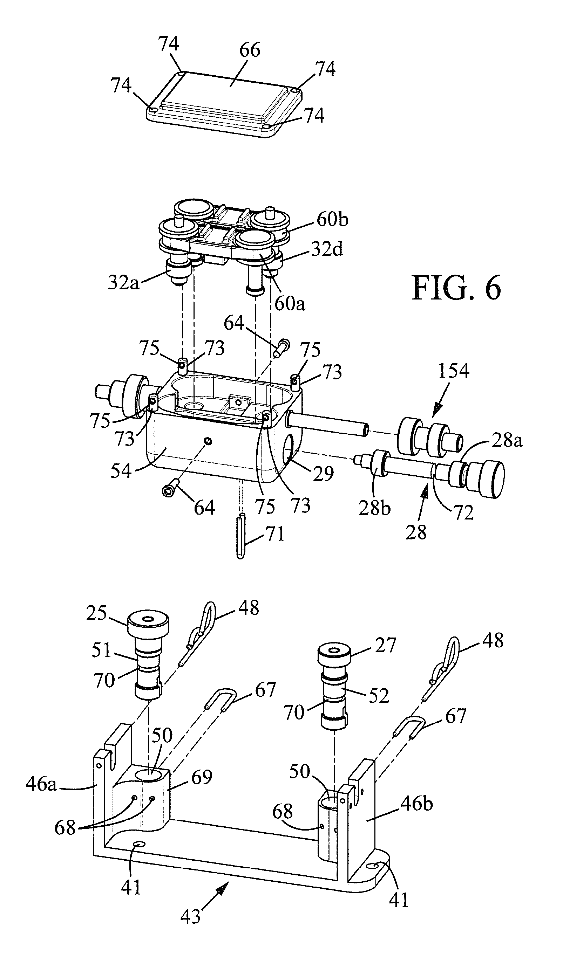

Dans un mode de réalisation particulier, comme représenté en particulier sur la figure 6, le système permet une utilisation répétée simple et peu coûteuse. En particulier, une majeure partie du système est disposée à l'abri d'une barrière stérile, comme expliqué ci-dessus, et ne nécessite donc pas d'opérations de stérilisation répétées complexes et coûteuses. En ce qui concerne la partie réduite du système destinée à être en contact avec le patient via un organe tubulaire souple médical allongé, elle est constituée exclusivement de pièces consommables, de pièces stérilisables, et de pièces d'assemblage rapide, elles mêmes stérilisables. En particulier, dans l'exemple présenté de module d'entraînement, seules les courroies sont réalisées comme des pièces consommables. En ce qui concerne les pièces d'assemblage, on peut par exemple prévoir des pièces à déformation élastique qui maintiennent ensemble les pièces stérilisables, le cas échéant avec possibilité de mouvement les unes par rapport aux autres. On peut par exemple faire référence au dispositif amovible formé de goupilles 48, déjà décrit plus haut en relation avec la figure 3, dont un premier bras se glisse dans une ouverture adéquate ménagée dans un bras 46a, 46b de la base et définit une partie du palier de rotation pour l'équipage mobile, et dont un deuxième bras est déformé élastiquement pour la rétention. A titre d'autre exemple, des broches 67 en forme de U ont leurs deux bras parallèles insérés dans des ouvertures correspondantes 68 ménagées dans les cylindres 69 de rétention des arbres de rotation ou de translation 51, 52. Ces arbres comportent chacun une gorge 70 qui coopère avec chacun des bras de la broche 67 correspondante afin d'empêcher un déplacement de l'arbre par rapport au cylindre le long de sa direction d'extension, tout en autorisant sa rotation.

Une broche 71 similaire est prévue pour coopérer avec une gorge 72 ménagée dans l'arbre 28, afin d'empêcher la sortie de l'arbre 28 hors de son cylindre de réception.

Des systèmes similaires peuvent être prévus pour maintenir le couvercle 66 sur la partie de fond 54 de l'équipage mobile. Ainsi la partie de fond 54 peut comporter, en surface supérieure, quatre pions 73. Le couvercle 66 est inséré sur ces pions par l'intermédiaire d'ouvertures 74 correspondantes disposées en chaque coin. Les pions 73 sont eux-mêmes pourvus d'ouvertures traversantes 75 alignées deux à deux, aptes à recevoir un bras d'une goupille (non représentée) similaire aux goupilles 48 décrites ci-dessus.

La fixation de la base 43 sur son support peut se faire d'une manière similaire, en retenant des tétons du support passant à travers les alésages 41 de la base 43 avec des moyens de rétention élastiques déformables.

Ainsi, les pièces d'assemblage et les pièces assemblées sont des pièces dites « stérilisables » en ce qu'elles sont nettoyables facilement en leur totalité, sans présenter de recoin ou de zone inaccessible au nettoyage.

Un exemple d'utilisation du dispositif représenté sur les figures est décrit ci-après. Il est à noter qu'à la figure 2, la quasi-totalité du cathéter est entrée à l'intérieur du patient. Un chirurgien ponctionne une artère, par exemple l'artère fémorale au niveau du pli inguinal et met en place un court tuyau comportant une valve réalisant une porte d'accès entre l'extérieur et l'artère, communément appelé désilet. L'enrouleur/dérouleur de la figure 2 est placé à proximité du patient, et est relié à l'ordinateur 4. L'enrouleur/dérouleur 5 contient déjà un liquide de conservation dans lequel baigne, dans le premier système, un cathéter, et dans le deuxième système un guide insérable dans le cathéter. En ouvrant au maximum le mécanisme de réglage 34 du premier système, le cathéter peut être déplacé jusqu'à être inséré manuellement par le chirurgien à travers le désilé dans l'artère. Puis, depuis l'ordinateur 4, le chirurgien commande le moteur électrique de translation du deuxième système pour guider le guide 37, à travers la sortie 18, à l'intérieur du cathéter 6, jusqu'à ce que la première extrémité du guide arrive, à l'intérieur du patient, au niveau de la première extrémité du cathéter. Pendant cette opération, la source de rayons X 8 peut émettre des rayonnements qui n'ont pas d'influence sur le chirurgien 2, et l'image par le détecteur 10 peut être affichée sur l'écran 11 de l'ordinateur.

Pour que l'extrémité du cathéter atteigne l'emplacement d'intérêt à l'intérieur du corps du patient 1, le chirurgien 2 commande, depuis l'ordinateur 4, les fonctions suivantes :
- translation du guide : par activation du moteur électrique de translation 26 du deuxième système, qui entraîne en rotation un ensemble de pièces d'entraînement, notamment la bague d'engrenage 22 de translation, l'engrenage intermédiaire 30, l'arbre 28, les roues dentées 32a et 32b et de ce fait, les poulies d'entraînement 31a à 31d et donc les courroies 60a et 60b qui génèrent une translation du guide le long de sa direction d'élongation à l'intérieur du cathéter 6,
- la rotation du guide autour de l'axe longitudinal du guide, en commandant l'actionnement du moteur électrique de rotation 24 du deuxième système, et par là un ensemble de pièces d'entraînement, notamment l'engrenage de rotation 25 et la bague d'engrenage de rotation 23 qui entraîne en rotation l'ensemble de l'équipage mobile et du guide par rapport aux paliers 20a et 20b du deuxième système, et par là celle du guide maintenu sur l'équipage par les courroies 60a et 60b,
- la translation du cathéter le long de sa direction d'élongation de cathéter par la commande similaire à la commande de translation du guide décrit précédemment, (du fait de la liaison de la deuxième extrémité du cathéter au dispositif de fixation 35, cette translation entraîne la rotation libre du deuxième système autour de l'axe Z par rapport au premier récipient), et
- la rotation du cathéter, commandée comme décrit précédemment pour le guide, le dispositif de fixation 35 autorisant le fait que le cathéter 6 tourne autour de son axe d'élongation sans influer sur le guide 37 ni le deuxième récipient.

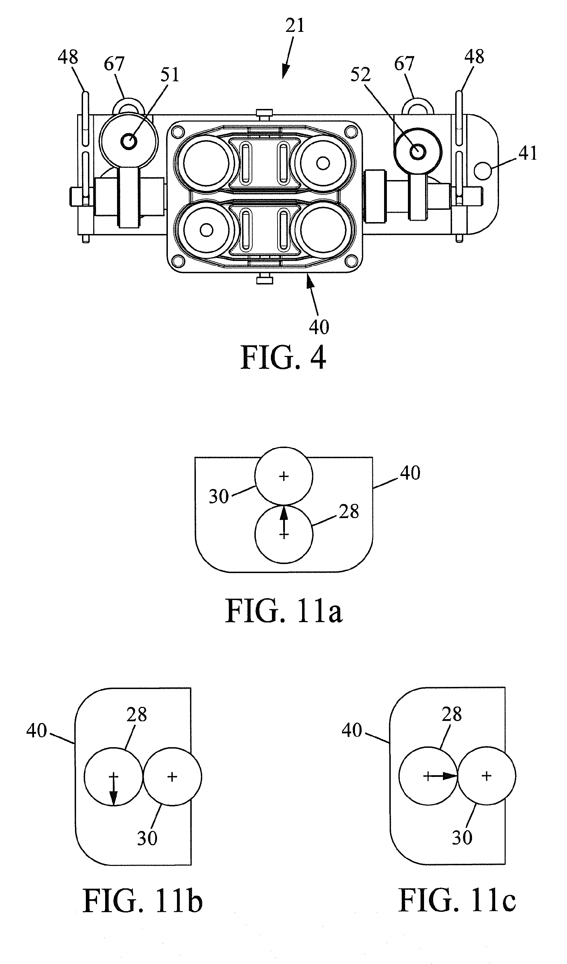

En pratique, du fait de l'utilisation d'engrenages, les commandes de translation et de rotation sont partiellement liées. La Fig. 11a représente une position de départ dans le référentiel de la pièce. Si seul le moteur de rotation est commandé, l'équipage mobile 40 va tourner autour de l'axe X comme représenté. Du fait de l'engrènement entre les roues dentées 30 et 28, si la roue dentée 30 est maintenue fixe (i.e. le moteur de translation n'est pas commandée), la roue dentée 28 va également tourner autour de son axe. Ceci est illustré à la Fig. 11b par une flèche repère indiquant une dent de référence de l'engrenage 28.

Ainsi, la commande de rotation génère une rotation de l'arbre 28 dans le référentiel de l'équipage mobile, et par conséquent une rotation des courroies (une translation du cathéter).

Pour générer un mouvement de pure rotation du cathéter, on applique donc, simultanément à la commande de rotation, une commande de translation adaptée pour contrebalancer ce déplacement. Les deux commandes sont appliquées selon un rapport prédéterminé (caractérisé par les engrenages entrant en jeu). Un mouvement de rotation pure est généré lorsque la dent repère de la roue dentée 28 reste pointée dans une direction constante dans le repère de l'équipage mobile, comme représenté sur la Fig. 11c.

Le cas échéant, la détection des mouvements des engrenages est utilisée pour adapter ce rapport.

Par contre, en cas d'application d'une commande du moteur de translation, aucune rotation n'est générée dans le module, et un mouvement de translation pure du cathéter est généré.

On peut ainsi prévoir que dans certains modes de réalisation, on peut commander conjointement la translation du guide et du cathéter, par une commande simultanée des deux moteurs correspondants.

Les commandes à la fois de translation et de rotation peuvent être bien entendu effectuées dans un sens ou dans l'autre, pour déplacer le guide et le cathéter jusqu'au site, où les en ramener.

Une rotation du deuxième récipient par rapport au premier récipient peut être obtenue en entraînant en rotation le plateau 39 par un moteur électrique 75 spécialement dédié connecté sous le plateau. Ce moteur dédié peut en particulier être utilisé pour guider le plateau après utilisation.

En outre, dans un mode de réalisation comprenant également l'insertion d'un cathéter interventionnel, destiné à apporter une certaine fonction au site, les opérations et variantes décrites ci-dessus peuvent également être mises en place pour le troisième système.

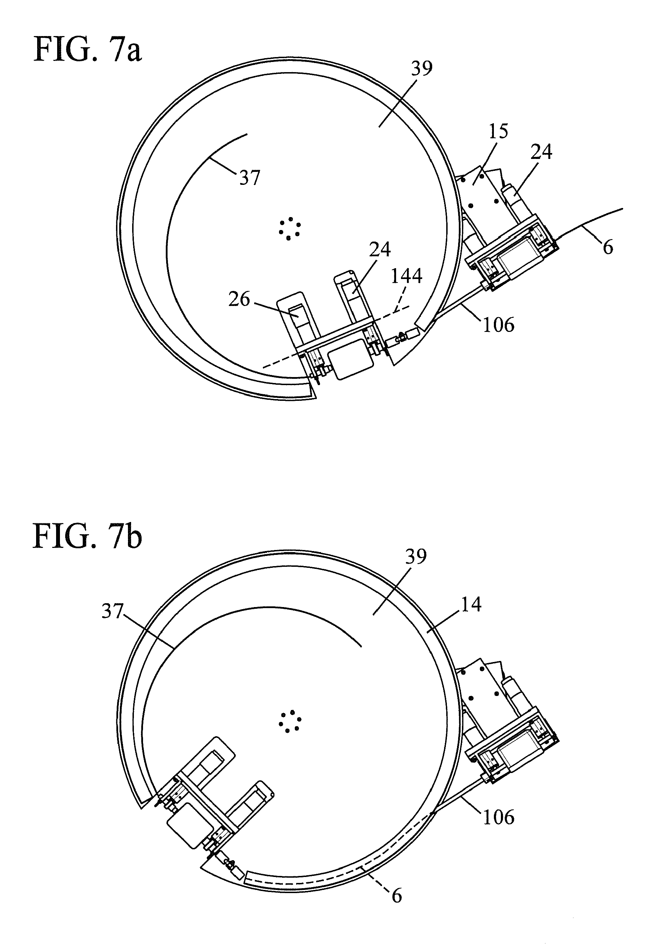

Ainsi, la figure 7a est une vue de dessus du système dans une position où le cathéter est quasiment entièrement entré dans le corps du patient.

Comme on le voit sur la figure 7b, lorsqu'on veut faire sortir une partie du cathéter du corps du patient, outre qu'on applique un mouvement de translation le long de la direction opposée à la direction X à l'aide du système d'entraînement, on fait appliquer une rotation, à l'aide du moteur 75 du plateau 39, afin que le cathéter 6 rentre à l'intérieur du récipient 14. Pour entraîner le cathéter en translation, on applique donc à la fois une commande au moteur de translation du cathéter, et une commande au moteur 75 du plateau, selon un rapport prédéterminé, afin que la longueur de déplacement du cathéter corresponde exactement à la longueur de sortie/entrée du cathéter dans le tube 14.

Par ailleurs, si on génère un mouvement de translation du cathéter par rapport au patient, comme expliqué ci-dessus, par commande du moteur de translation de cathéter et du moteur 75 du plateau 39, la rotation du plateau 39 entraîne la translation du guide par rapport au patient, si le deuxième système d'entraînement est inactif pendant cette étape. Si on souhaite au contraire que le guide reste immobile dans le patient pendant cette étape, on commande le moteur de translation du guide pour faire translater celui-ci par rapport au deuxième système, selon un rapport prédéterminé avec la commande en translation du cathéter.

Ainsi, les différentes motorisations à appliquer aux différents moteurs pour obtenir les différents mouvements unitaires sont résumés dans le tableau ci-après :

| | Guide | | Cathéter | | Plateau |
|---|---|---|---|---|---|
| | Translation | Rotation | Translation | Rotation | Rotation |
| Translation guide seul | X | | | | |
| Rotation guide seul | X | X | | | |
| Translation cathéter seul | X | | X | | X |
| Rotation cathéter seul | | | X | X | |
| Translation cathéter et guide | | | X | | X |
| Rotation cathéter et guide | X | X | X | X | |

Les motoréducteurs sont munis de codeurs d'impulsions donnant les déplacements. Ces déplacements sont mesurés par tout moyen approprié.

Dans le cas de la mise en oeuvre d'un troisième système, les enseignements ci-dessus seront adaptés.

Ainsi, de manière générale, le système de cathétérisme robotisé comprend :
- une unité centrale,
- au moins une première, une deuxième et une troisième lignes de commande adaptées respectivement pour envoyer une commande à un premier, un deuxième et un troisième moteurs d'entraînement d'un même cathéter (les moteurs de translation et de rotation et le moteur du plateau),
- dans lequel les première et deuxième lignes de commande (translation et plateau) sont commandées selon un premier rapport prédéfini pour commander un mouvement de translation pure du cathéter le long de son axe, et dans lequel les première et troisième lignes de commande (translation et rotation) sont commandées selon un deuxième rapport prédéfini pour commander un mouvement de rotation pure du cathéter le long de son axe.

Pour générer un mouvement de rotation pure d'un organe, on commande aussi le moteur de translation du système d'entraînement associé à cet organe selon un rapport prédéterminé.

Pour générer un mouvement de translation différentielle des deux organes, on commande les moteurs de translation des 2 systèmes d'entraînement et le moteur de plateau selon des rapports prédéterminés.

Après utilisation, le démontage du système peut s'effectuer comme suit :
- on désassemble la base 43 de son support en retirant les systèmes d'assemblage rapide servant à cet assemblage,
- on retire les goupilles 48, de manière à désassembler la base 43 de l'équipage mobile 40, en faisant sortir l'équipage mobile hors des fentes 47,
- on retire les dispositifs de fixation rapides 67, de manière à désassembler les arbres de rotation et de translation 51, 52 de la base 43,
- on retire le manchon 154 en le faisant coulisser le long de l'arbre aval 149,
- on retire le couvercle 66 de la partie de fond 54, en retirant les goupilles passant à travers les orifices 75 de la partie de fond,
- on retire de la partie de fond 54 les poussoirs 63,
- on retire de la partie de fond 54 les arbres 58a-58d portant, respectivement, les poulies 31a-31d et les courroies, et on retire les courroies de ces poulies,
- on retire l'arbre 28 de la partie de fond 54 par retrait de la goupille 74 et coulissement via l'orifice 29,
- on dévisse les vis de réglage 75.

Les éléments consommables peuvent être jetés, et les divers éléments non consommables sont des pièces de petites dimensions et formes simples, pouvant être stérilisées.

Les éléments consommables du robot, tel que le tube 14 contenant le cathéter retiré du corps du patient, ou le dispositif de liaison 35 peuvent également être démontés et jetés ou stérilisés selon les cas et les barrières stériles sont mises au rebus.

Pour une opération suivante, l'assemblage du système peut se faire, par un opérateur stérile, par une suite d'opérations inverses de la suite d'opérations décrites ci-dessus pour le démontage.

Le système de réglage 34 peut être réalisé de toute manière appropriée, tel qu'un système mécanique utilisant des vis 64 ou, en alternatives, un système piézo-électrique ou autre.

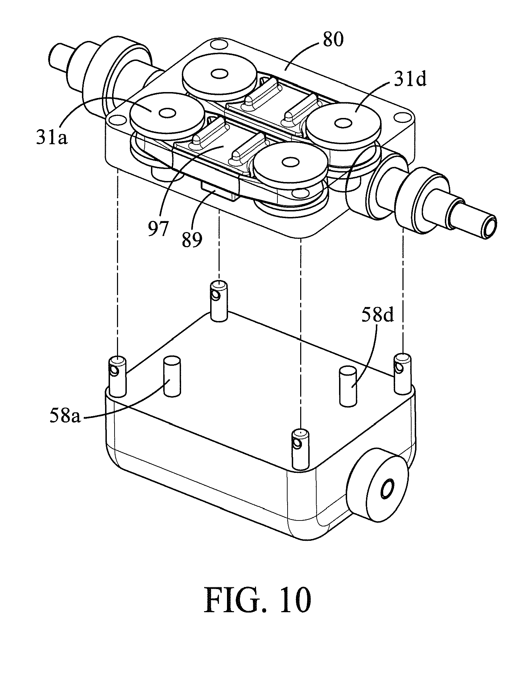

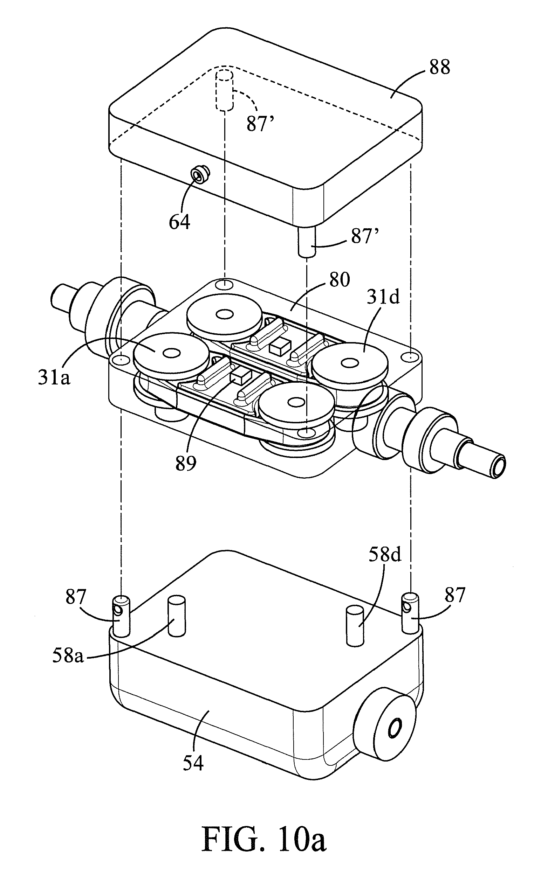

Bien que, dans le mode de réalisation présenté ci-dessus, certains éléments sont présentés comme étant indépendants, on pourra en variante prévoir d'assembler ou de pré-assembler certains de ces éléments les uns aux autres, si approprié. Par exemple, comme représenté sur la figure 10, on pourrait prévoir que les poulies 31a-31d sont pré-assemblées (de manière mobile) ensemble sur une base 80 commune assemblable sur des arbres d'entraînement 58a-58d prêts à assembler (le cas échéant de manière amovible) sur la partie de fond 54. Cette partie 80 comprenant les poulies et, le cas échéant, les courroies, pourrait être réalisée comme un consommable.

Une autre variante de réalisation est représentée sur la figure 10a. Deux broches d'extrémité 87 sont utilisées pour assembler la partie de fond 54 à la partie consommable 80, et une partie de couvercle 88 est assemblée à la partie consommable 80 de manière similaire, à l'aide de deux broches d'extrémité 87', par exemple fournies selon une diagonale opposée à celle des broches 87. La partie 80 est donc maintenue entre la partie de fond 54 et la partie de couvercle 88. La partie de couvercle 88 peut également comporter le système de réglage 34 des poussoirs. Ainsi, contrairement au mode de réalisation de la Fig. 10 où la zone d'entraînement 89 des poussoirs par la vis 64 (non montrée) est située sous le corps 97 du poussoir, dans la Fig. 10a, cette zone 89 est placée sur le dessus du corps de poussoir, de manière à interagir avec la vis 64 montée dans le couvercle 88.

Ainsi, selon un aspect, l'équipage mobile 40 comprend :
- une partie de fond 54 comprenant au moins un arbre d'entraînement, et
- une partie 80 comprenant au moins lesdites poulies. La partie 80 est assemblable par fixation rapide sur la partie de fond, la poulie motorisée coopérant avec l'arbre d'entraînement lors de cet assemblage.

Ainsi, dans les deux modes de réalisation représentés ci-dessus, l'axe autour duquel l'équipage mobile tourne par rapport à la base, et l'axe médian du cathéter à l'endroit où celui-ci est entraîné par les courroies, sont confondus, et parallèles à la direction longitudinale X.

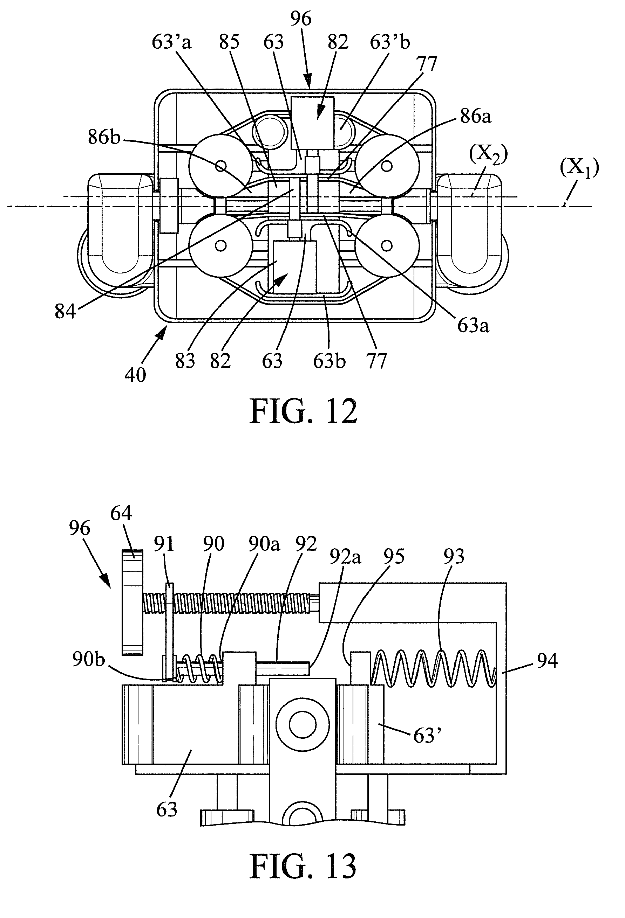

Selon un autre mode de réalisation, représenté sur la figure 12, on a représenté l'axe de rotation X1 et l'axe d'entraînement en translation X2 décalés l'un par rapport à l'autre selon une direction transversale à la direction longitudinale. L'axe d'entraînement en translation X2 est défini comme l'axe médian entre les deux surfaces d'entraînement 77 en usage, qui sont elles-mêmes définies par les surfaces des courroies. La position de l'axe 52 en usage est donc définie par la position des poussoirs 63. C'est-à-dire que le canal comprend une portion centrale 85 s'étendant selon l'axe X2 et des portions d'entrée 86a et de sortie 86b formant un angle non nul avec la direction longitudinale. Ainsi, on prévoit un système de latéralisation 96 qui définit la position de l'axe X2 par rapport à l'axe de rotation X1. La valeur du décalage entre les deux axes peut donc être réglable. Les poussoirs 63 ne servent pas uniquement à définir la largeur du canal, la force de serrage appliquée au cathéter, ou la tension des courroies, mais aussi le décalage des axes X1 et X2.

Ainsi, dans le mode de réalisation présenté, le poussoir inférieur 63 comporte une portion intérieure 63a et une portion extérieure 63b pouvant être fixée en une pluralité de positions distinctes selon l'axe transversal par rapport à la portion intérieure 63a. Cet ajustement permet de régler la tension de la courroie inférieure. Le poussoir supérieur 63 comporte une portion intérieure 63'a similaire à la portion intérieure 63a et une portion extérieure 63'b. La portion extérieure 63'b est ici indépendante de la portion intérieure 63'a, et réalisée par exemple sous la forme de deux galets tendeurs. Au besoin, ceux-ci peuvent être prévus mobiles pour que leur déplacement selon l'axe transversal permette d'ajuster la tension de la courroie. Le déplacement des deux portions intérieures dans des sens opposés permet d'une part d'adapter le système à la largeur de l'organe à entraîner, et d'autre part de régler la force de serrage appliquée à cet organe. Le déplacement des deux portions intérieures dans les mêmes sens permet de régler le décalage d'axe.

Lors de la rotation de l'équipage mobile 40 sur la base 43, ce décalage d'axe permet d'obtenir le déplacement de rotation souhaité de l'extrémité du cathéter à l'intérieur du patient.

Le cas échéant, le décalage d'axe pourra être fait varier au cours de l'utilisation du robot. Ainsi, on peut utiliser des dispositifs de motorisation 82 commandés à distance par l'utilisateur, pour déplacer chaque poussoir en position souhaitée. Un exemple de réalisation comprend un moto-réducteur 83 entraînant une vis sans fin 84 tournant dans un écrou fixé à chaque poussoir. D'autres variantes que celle représentée sont possibles, comme d'avoir les deux moto-réducteurs situés d'un même côté du canal, et/ou d'utiliser un vérin ou un micro-positionneur. Selon une autre variante, plutôt que de régler indépendamment la position de chaque poussoir, on pourra lier les deux mécanismes, de manière à disposer d'une commande pour la position de l'axe X2, et d'une autre commande pour l'écartement des poussoirs par rapport à cet axe (largeur du cathéter et serrage de celui-ci). Enfin, on notera que le décalage d'axe pourrait en variante être mis en oeuvre pour d'autres types de système d'entraînement en translation embarqués sur une plaquette rotative, tels que notamment un système présentant un ou plusieurs galet(s) d'entraînement présentant une surface d'entraînement non étendue.

Selon le mode de réalisation de la Fig. 13, on utilise un système de latéralisation 96 unique pour, premièrement, enserrer le cathéter avec une force prédéterminée (par exemple 6 Newtons) puis, lors d'une poursuite de l'actionnement de la commande du système, décaler l'axe d'entraînement X2 par rapport à l'axe de rotation X1. A titre d'exemple, on prévoit un premier ressort 90 (ou tout autre élément élastique adapté) s'étendant entre une première extrémité 90a sollicitant un premier poussoir 63, et une deuxième extrémité 90b sollicitant un coulisseau 91. Le coulisseau lui-même est monté mobile selon l'axe transverse de déplacement du poussoir, par exemple par l'intermédiaire de la vis 64. Le ressort 90 est par ailleurs porté par une tige 92 présentant une extrémité de butée 92a. Un deuxième ressort 93 (ou tout autre élément élastique adapté) est fourni entre le bâti 94 et le deuxième poussoir 63'. Le deuxième poussoir 63' est muni d'une surface de butée 95 faisant face à la surface de butée 92a de la tige.

L'actionnement de la vis 64 génère la translation du coulisseau 91 et ainsi le déplacement du premier poussoir 63 jusqu'à ce que l'extrémité de butée 92a de la tige vienne en butée sur la butée 95. Dans cette position, le cathéter est soumis à la force de serrage prédéterminée, selon la compression du ressort 90. La poursuite de l'actionnement de la vis 64 va causer la compression du ressort 93, et par conséquent le déplacement des premier et deuxième poussoirs en direction du bâti. Ainsi, la poursuite de l'actionnement de la vis 64 va causer le décalage d'axe X2.

On notera que la vis 64 peut être entraînée manuellement, ou de manière motorisée, par exemple par un moteur alimenté par batterie, ou encore mécaniquement à distance par un actionneur, dans une position prédéterminée du module (dite position de réglage), où l'actionneur se trouve dans une position apte à réalisée cet entraînement.

En variante de la vis 64, on peut utiliser d'autres systèmes, tels que des systèmes à crémaillère, par exemple.

Ainsi, on prévoit un module d'entraînement d'un organe médical souple allongé selon une première direction, comprenant une base et un équipage mobile monté rotatif sur ladite base autour d'un premier axe s'étendant selon la première direction, l'équipage mobile comportant un support,
dans lequel le module comprend :
- un canal défini dans le support, et s'étendant selon la première direction,
- de chaque côté du canal :
   . au moins un premier et un deuxième organe d'entraînement portés par le support, et présentant chacun une face adaptée pour coopérer avec l'organe médical souple,
dans lequel le canal comprend une portion selon un deuxième axe, parallèle au premier axe et décalé par rapport à celui-ci.

Tant que possible, on privilégiera un système compatible avec les principes de montage/démontage simple et de stérilisation (ou réalisation comme consommables) énoncés au-dessus pour les deux premiers modes de réalisation.

## Revendications

1. Module d'entraînement d'un organe médical souple allongé selon une première direction (X), comprenant une base (43) et un équipage mobile (40) monté rotatif sur ladite base autour de la première direction (X), l'équipage mobile comportant un support (54),
**caractérisé en ce que** le module comprend :
- un canal (59) défini dans le support (54), et s'étendant selon la première direction,
- de chaque côté du canal :
. au moins une première et une deuxième poulies (31a-31d) comportant une surface d'entraînement (77), et portées par le support (54),
. une bande (61a-61b) allongée comprenant une première face (78a) et une deuxième face (78b) opposée, la première face coopérant avec la surface d'entraînement des poulies, la deuxième face adaptée pour coopérer avec l'organe médical souple, la bande étant tendue par les poulies avec une portion allongée s'étendant dans le canal selon la première direction (X),
au moins une des poulies (31a, 31d) étant motorisée.

2. Module d'entraînement selon la revendication 1, comprenant en outre un dispositif de réglage (34) placé entre la première et la deuxième poulie, comprenant une surface tendeuse (81) coopérant avec la première face (78a) de la bande, et mobile par rapport au canal le long d'une direction d'ajustement transverse à la première direction.

3. Module d'entraînement selon la revendication 1 ou 2, dans lequel la première face (78a) de la bande présente des dents d'entraînement et dans lequel la surface d'entraînement (77) de la poulie motorisée présente des dents complémentaires auxdites dents d'entraînement.

4. Module d'entraînement selon l'une quelconque des revendications 1 à 3, dans lequel l'équipage mobile comprend :
- une partie de fond (54) comprenant au moins un arbre d'entraînement, et
- une partie consommable dans laquelle ledit canal est défini, la partie consommable comprenant au moins lesdites poulies, la partie consommable étant assemblable sur la partie de fond, la poulie motorisée coopérant avec ledit arbre d'entraînement.

5. Module d'entraînement selon au moins l'une des revendications 1 à 4, dans lequel l'équipage mobile (40) est monté rotatif sur la base autour d'un premier axe, et dans lequel le canal (59) comprend une portion définie dans le support (54) selon un deuxième axe, parallèle au premier axe et décalé par rapport à celui-ci.

6. Module d'entraînement selon la revendication 5, comprenant un dispositif de latéralisation (96) adapté pour régler un décalage du premier et du deuxième axe.

7. Module d'entraînement selon la revendication 6, dans lequel le dispositif de latéralisation (96) comporte une commande (64) adaptée pour, lors d'une première partie de course d'entraînement, causer un enserrement de l'organe médical, et lors d'une deuxième partie de course d'entraînement suivant la première partie, régler ledit décalage du premier et du deuxième axe.

8. Module d'entraînement selon au moins l'une des revendications 1 à 7, comprenant un ensemble de pièces d'entrainement (51,23 ;52,154,28,58a-58d) coopérant entre elles pour transmettre un mouvement d'un moteur à la poulie motorisée (31a-31d).

9. Module d'entraînement selon au moins l'une des revendications 1 à 8, constitué desdites bandes (61a-61b), de pièces stérilisables, et de pièces d'assemblage (48, 67,71) amovibles à déformation élastique adaptées pour maintenir ensemble les pièces stérilisables avec liberté de mouvement les unes par rapport aux autres.

10. Système robotisé comprenant :
- un module d'entraînement selon au moins l'une des revendications 1 à 9,
- un récipient (14, 17) adapté pour contenir un organe médical souple allongé en condition aqueuse stérile, et en communication avec ledit canal,
- un moteur (24,26) coopérant avec la poulie motorisée.

11. Système robotisé selon la revendication 10, comprenant en outre une barrière stérile (44), le moteur coopérant avec la poulie motorisée à travers la barrière stérile.

12. Système robotisé selon la revendication 10 ou 11, comprenant au moins trois moteurs adaptés pour générer différents mouvements à au moins un organe médical souple allongé, et comprenant une unité centrale adaptée pour commander simultanément au moins deux moteurs selon des rapports prédéterminés.
